# EUROPÄISCHE PATENTANMELDUNG

(11) **EP 3 441 942 A1**
(43) Veröffentlichungstag der Anmeldung: **13.02.2019**
(21) Anmeldenummer: 17185956.4
(22) Anmeldetag: 11.08.2017
(51) Int. Cl.: G06T 11/00

(54) **VERFAHREN ZUR ERSTELLUNG EINES VOLUMENBILDES UND RÖNTGENBILDGEBUNGSSYSTEM**

(71) Anmelder: Siemens Healthcare GmbH, 91052 Erlangen (DE)
(72) Erfinder: Hornung, Oliver, 91077 Dormitz (DE); Kowarschik, Markus, 90408 Nürnberg (DE)

(57) **Zusammenfassung**

Verfahren zur Erstellung eines Volumenbildes eines sich bewegenden Untersuchungsobjekts in einem ausgewählten Bewegungszustand aus einer Serie von Projektionsbildern, welche während einer Rotationsbewegung eines Aufnahmesystems um das Untersuchungsobjekt mit jeweils einer Vielzahl von Projektionsbildern aus unterschiedlichen Projektionsrichtungen aufgenommen wurde, mit den Schritten Bereitstellung der Serie von Projektionsbildern (P₁, P₂ ...P_{N}), Auswahl eines ersten Bewegungszustands insbesondere durch Auswahl eines ersten Projektionsbildes, das den ersten Bewegungszustand abbildet, Bereitstellung oder Ermittlung einer Konsistenz- oder Redundanzbedingung, Identifikation weiterer Projektionsbilder, die den ersten Bewegungszustand abbilden, durch Anwendung der Konsistenz- oder Redundanzbedingung auf die Projektionsbilder der Serie, und Rekonstruktion der identifizierten Projektionsbilder und des ersten Projektionsbildes zu einem rekonstruierten Volumenbild

## Beschreibung

Die Erfindung betrifft ein Verfahren zur Erstellung eines Volumenbildes eines Untersuchungsobjekts in einem gewünschten Bewegungszustand gemäß dem Patentanspruch 1 sowie ein Röntgenbildgebungssystem zur Durchführung eines derartigen Verfahrens gemäß dem Patentanspruch 8.

Zeitaufgelöste 3D-Röntgenbildgebung (4D-Bildgebung) mit hohem Weichteilkontrast mittels eines C-Bogen-Röntgenbildgebungssystems ist derzeit allenfalls in Spezialfällen möglich, beispielsweise unter der Annahme sowohl periodischer als auch hinreichend glatter Bewegungsfelder. Zunächst wird eine Serie von Projektionsdatensätzen eines sich im Allgemeinen periodisch bewegenden Untersuchungsobjekts aufgenommen, während der C-Bogen um das Untersuchungsobjekt rotiert, also aus einer Vielzahl von Projektionsrichtungen des C-Bogens in Bezug auf das Untersuchungsobjekt. Die Serie umfasst dabei im Allgemeinen zumindest einen halben Rotationslauf um das Untersuchungsobjekt.

Bei der Rekonstruktion der Projektionsdatensätze besteht insbesondere das Problem, dass das Untersuchungsobjekt sich in abfolgenden Projektionsbildern in unterschiedlichen Bewegungszuständen befindet. Um die entsprechenden übereinstimmenden Bewegungszustände zu Volumenbildern rekonstruieren zu können, muss eine Erfassung der Bewegungszustände und Zuordnung zu den jeweiligen Projektionsbildern vorgenommen werden. Dies ist mit hohem Aufwand verbunden und insbesondere bei schnellen Bewegungen sehr ungenau. Die Erfassung von Bewegungszuständen wird z.B. mittels optischem Tracking bei Atembewegungen oder EMT bei Herzschlag durchgeführt. Ein weiteres Problem besteht darin, dass bei bekannten C-Bögen keine Endlosrotationen möglich sind, sondern Vorwärts-Rückwärts-Rotationen in Abfolge, was zu einer Einschränkung des Winkelbereichs und eingeschränkter Dynamik durch mechanische Instabilitäten des C-Bogens führt. Letzteres Problem kann z.B. durch die Einführung eines Schleifringes an der Aufhängung des C-Bogens gelöst werden, so dass Endlosrotationen möglich sind, siehe z.B. auch die gleichzeitig angemeldete Patentanmeldung "Verfahren zur Aufnahme eines Bilddatensatzes mit einem Röntgenbildgebungssystem und Röntgenbildgebungssystem".

Es ist Aufgabe der vorliegenden Erfindung, ein Verfahren bereitzustellen, welches eine einfache zeitaufgelöste 3D-Rekonstruktion aus einer Serie von Projektionsbildern ohne ein Hinzuziehen von zusätzlichen Messungen von Bewegungszuständen ermöglicht; des Weiteren ist es Aufgabe der Erfindung, ein für die Durchführung des Verfahrens geeignetes Röntgenbildgebungssystem bereitzustellen.

Die Aufgabe wird erfindungsgemäß gelöst durch ein Verfahren zur Erstellung eines Volumenbildes eines Untersuchungsobjekts in einem gewünschten Bewegungszustand gemäß dem Patentanspruch 1 und von einem Röntgenbildgebungssystem gemäß dem Patentanspruch 8. Vorteilhafte Ausgestaltungen der Erfindung sind jeweils Gegenstand der zugehörigen Unteransprüche.

Bei der vorliegenden Erfindung geht es um die tomographische Rekonstruktion zeitaufgelöster volumetrischer Bilder mit hohem Weichteilkontrast, entweder ganz ohne Verwendung von Kontrastmittel (z.B. die Darstellung der Bronchien während endobronchialer oder perkutaner Lungeninterventionen) oder bei (quasi-)stationärer Füllung mit Kontrastmittel (z.B. die Darstellung der Leber während onkologischer Interventionen), also ohne signifikante Kontrastmitteldynamik.

Das erfindungsgemäße Verfahren zur Erstellung eines Volumenbildes eines sich bewegenden Untersuchungsobjekts in einem ausgewählten Bewegungszustand aus einer Serie von Projektionsbildern, welche während einer Rotationsbewegung, insbesondere aus zumindest einem halben Rotationslauf, eines Aufnahmesystems um das Untersuchungsobjekt mit jeweils einer Vielzahl von Projektionsbildern aus unterschiedlichen Projektionsrichtungen aufgenommen wurde, umfasst die folgenden Schritte: Bereitstellung der Serie von Projektionsbildern, Auswahl eines ersten Bewegungszustands insbesondere durch Auswahl eines ersten Projektionsbildes welches den ersten Bewegungszustand abbildet, Bereitstellung oder Ermittlung einer Konsistenz- oder Redundanzbedingung, Identifikation weiterer Projektionsbilder, die den ersten Bewegungszustand abbilden, durch Anwendung der Konsistenz- oder Redundanzbedingung auf die Projektionsbilder der Serie, und Rekonstruktion eines Volumenbilds aus den identifizierten Projektionsbildern und dem ersten Projektionsbild.

Die Serie von Projektionsbildern ist z.B. während mindestens einem halben (180°) oder mehr (z.B. 270°) oder auch einem ganzen oder mehr, z.B. zwei, Rotationsumläufen eines Aufnahmesystems um das Untersuchungsobjekt aufgenommen worden bzw. wird aufgenommen. Ein < 180° Umlauf ist auch möglich, aber nur unter bestimmten Bedingungen sinnvoll, im Allgemeinen sind mindestens 180° plus Öffnungswinkel des Röntgenstrahls notwendig. Die Bewegung des Untersuchungsobjekts kann, muss aber nicht periodisch sein. Periodische oder teilperiodische Bewegungen wie z.B. Atemzyklen oder Herzschlag verbessern die Qualität des Rekonstruktionsergebnisses. Im periodischen Fall sollten wenn möglich mindestens zwei, bevorzugt eine Vielzahl von Bewegungszyklen aufgenommen sein, damit mindestens zwei übereinstimmende Bewegungszustände vorhanden sind.

Die Auswahl eines Bewegungszustandes ist z.B. manuell durch eine manuelle Eingabe eines Benutzers durchführbar, also ein Benutzer wählt aus der Serie ein Projektionsbild des gewünschten Bewegungszustands aus. Es kann auch automatisch ein bestimmter Bewegungszustand (z.B. maximale Inhalation bei Atembewegung, erstes Bild der Serie ...) vorgegeben sein. Die Ermittlung einer Konsistenz- oder Redundanzbedingung kann z.B. wie in dem Artikel Rigid Motion Compensation in Interventional C-arm CT Using Consistency Measure on Projection Data, R. Frysch und G. Rose, MICCAI 2015, Part I, LNCS 9349, pp. 298-306, 2015 beschrieben, berechnet werden. Aus dem Stand der Technik sind verschiedene Möglichkeiten der Berechnungen solcher Konsistenz- oder Redundanzbedingungen bekannt.

Es kann auch eine Konsistenz- oder Redundanzbedingung bereitgestellt werden, welche z.B. zuvor gespeichert wurde. Mittels der Konsistenz- oder Redundanzbedingung werden dann durch Filterung alle Projektionsbilder der Serie von Projektionsbildern ermittelt, welche im selben Bewegungszustand aufgenommen wurden. Dies sind mindestens zwei, bevorzugt eine Vielzahl von Projektionsbildern aus unterschiedlichen Projektionsrichtungen, da ein Übereinstimmen der Projektionsrichtung sehr unwahrscheinlich ist. Anschließend wird aus dem ursprünglich ausgewählten und den mittels der Konsistenz- oder Redundanzbedingung identifizierten Projektionsbildern ein Volumenbild in einem bestimmten Bewegungszustand rekonstruiert. Das erfindungsgemäße Verfahren ist rein bildbasiert. Das bedeutet, dass keine weiteren Maßnahmen neben der eigentlichen Bildakquisition ergriffen werden müssen, um eine zeitaufgelöste Serie von Volumendatensätzen errechnen zu können. Insbesondere ist keine separate Hardware zur Erfassung des Bewegungszustands des Untersuchungsobjekts (Patient) erforderlich, wie bspw. ein Gurt zur Erfassung der Atembewegung bei Leberaufnahmen. Das bedeutet, dass das Verfahren besonders schnell, einfach und aufwandsarm durchgeführt werden kann und auch noch nachträglich, wenn die Serie von Projektionsbildern bereits vorliegt.

Nach einer Ausgestaltung der Erfindung wird die Rekonstruktion mittels eines iterativen Rekonstruktionsverfahrens, insbesondere eines ART (algebraic reconstruction technique) oder SART (Simultaneous Algebraic Reconstruction Technique) durchgeführt. Derartige Verfahren sind aus der Literatur bekannt. Sie sind besonders geeignet, da sie keine äquidistante Verteilung der Projektionsbilder entlang der Trajektorie des Rotationsumlaufs des Aufnahmesystems erfordern, wie dies z.B. bei herkömmlichen analytischen Rekonstruktionsverfahren (z.B. der FDK-Methode) idealerweise der Fall ist, denen eine numerische Integration zugrunde liegt.
Nach einer weiteren Ausgestaltung der Erfindung weist die Konsistenz- oder Redundanzbedingung einen vorgebbaren Toleranzschwellwert bezüglich des Bewegungszustands auf. Das bedeutet, dass auch Projektionsbilder mit um höchstens den Toleranzschwellwert vom Bewegungszustand abweichenden Bewegungszuständen ermittelt und für die Rekonstruktion verwendet werden. Hierdurch stehen mehr Projektionsbilder zur Verfügung, was ein qualitativ besseres Rekonstruktionsergebnis bewirkt, welches jedoch teilweise von Bewegungsunschärfe begleitet werden kann.

Nach einer weiteren Ausgestaltung der Erfindung werden mehrere Volumenbilder eines Untersuchungsobjekts in mehreren, insbesondere aufeinander abfolgenden, ausgewählten Bewegungszuständen ermittelt. So kann z.B. das Untersuchungsobjekt auch in einer ganzen Reihe aufeinanderfolgender Bewegungszustände rekonstruiert werden, z.B. bei Atembewegungen vom Zustand der maximalen Exhalation hin zum Zustand der maximalen Inhalation. In vorteilhafter Weise können die Volumenbilder zu einem zeitlich abhängigen Bilddatensatz, z.B. einer Videosequenz, zusammengefügt werden.

Zur Durchführung des Verfahrens ist ein Röntgenbildgebungssystem geeignet, aufweisend ein Aufnahmesystem mit einem Röntgendetektor und einer Röntgenquelle, die an einer Halterung angeordnet sind, welche Halterung dazu ausgebildet ist, um ein Untersuchungsobjekt zu rotieren und dabei eine Vielzahl von Projektionsbildern aus unterschiedlichen Projektionsrichtungen aufzunehmen, sowie aufweisend eine Systemsteuerung zur Ansteuerung des Röntgenbildgebungssystems, eine Recheneinheit zur Ermittlung der Konsistenz- oder Redundanzbedingung und ein Bildbearbeitungssystem zur Bearbeitung von Projektionsbildern und zur Rekonstruktion der Projektionsbilder zu Volumenbildern. Insbesondere wird die Halterung von einem C-Bogen gebildet und ist der C-Bogen mittels einer Schleifringkonstruktion aufgehängt, so dass eine Endlosrotation des C-Bogens ausführbar ist. Zweckmäßigerweise weist das Röntgenbildgebungssystem eine Speichereinheit zur Speicherung von Bilddaten und/oder Berechnungsdaten auf. Insbesondere weist das Röntgenbildgebungssystem eine Eingabeeinheit zur Entgegennahme einer Benutzereingabe auf. Alternativ kann auch ein Computertomographiesystem verwendet werden.

Idealerweise weist das Röntgenbildgebungssystem bei seinen Rotationen ein besonders glattes Bewegungsfeld bzw. eine in Bezug auf Abweichungen optimierte Mechanik auf.

Die Erfindung sowie weitere vorteilhafte Ausgestaltungen gemäß Merkmalen der Unteransprüche werden im Folgenden anhand schematisch dargestellter Ausführungsbeispiele in der Zeichnung näher erläutert, ohne dass dadurch eine Beschränkung der Erfindung auf diese Ausführungsbeispiele erfolgt. Es zeigen:
- FIG 1: ein Ablauf eines Verfahrens gemäß der Erfindung;
- FIG 2: ein dem Ablauf des Verfahrens in FIG 1 entsprechendes Diagramm mit resultierenden Projektionsbildern und Volumenbildern; und
- FIG 3: eine Ansicht eines C-Bogen-Röntgenbildgebungssystems mit einem Schleifring.

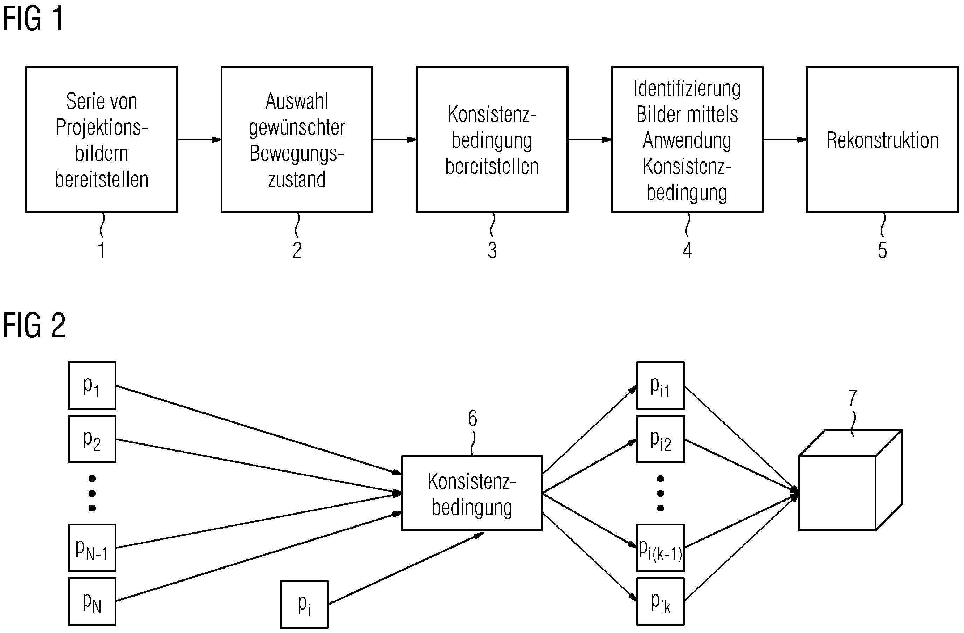

In der FIG 1 ist eine Abfolge eines erfindungsgemäßen Verfahrens mit fünf Schritten gezeigt, FIG 2 zeigt die entsprechenden aus den jeweiligen Schritten ermittelten Projektionsbilder bzw. Volumenbilder.

In einem ersten Schritt 1 wird eine Serie von Projektionsbildern P₁, P₂, ... P_{N-1}, P_{N} eines Untersuchungsobjekts aufgenommen oder alternativ bereitgestellt. Diese Serie kann z.B. während einer Rotation eines Aufnahmesystems um das Untersuchungsobjekt aufgenommen werden, muss jedoch nicht zwingend einen bestimmten Winkelabschnitt oder eine bestimmte Anzahl von Umläufen umfassen. Das Untersuchungsobjekt kann dabei z.B. eine zyklische Bewegung (Atemzyklus, Herzschlag) oder auch nichtzyklische Bewegung durchlaufen. Die Aufnahme der Serie P₁, P₂, ... P_{N-1}, P_{N} wird mittels eines später noch zu beschreibenden Röntgenbildgebungssystems durchgeführt, sie kann auch bereits vor dem Beginn des Verfahrens aufgenommen und dann im ersten Schritt 1 bereitgestellt, also z.B. aus einem Speicher abgerufen werden.

In einem zweiten Schritt 2 wird eine Auswahl bezüglich des gewünschten ersten Bewegungszustandes des Untersuchungsobjekts getroffen, wobei dies i.A. durch Auswahl eines ersten Projektionsbildes Pᵢ, welches den ersten Bewegungszustand des Untersuchungsobjekts abbildet, aus der Serie P₁, P₂, ... P_{N-1}, P_{N} durchgeführt wird. So kann z.B. eine Bedienperson eine Eingabe durchführen und das erste Projektionsbild Pᵢ auswählen, z.B. direkt durch Anklicken des entsprechenden Projektionsbildes. Alternativ kann auch der entsprechende Bewegungszustand selbsttätig, z.B. bei Atembewegung maximale Exhalation oder Inhalation ausgewählt werden. Für die Eingabe durch eine Bedienperson kann eine Eingabeeinheit (Tastatur, Touchbildschirm etc.) verwendet werden.

In einem dritten Schritt 3 wird die Konsistenz- oder Redundanzbedingung 6 bereitgestellt oder ermittelt. Die mathematische Herleitung einer solchen Konsistenz- oder Redundanzbedingung 6 kann entsprechend der Methode in dem Artikel Rigid Motion Compensation in Interventional C-arm CT Using Consistency Measure on Projection Data, R. Frysch und G. Rose, MICCAI 2015, Part I, LNCS 9349, pp. 298-306, 2015, durchgeführt werden. Aus dem Stand der Technik sind weitere Möglichkeiten zur Ermittlung von Konsistenz- oder Redundanzbedingungen bekannt. Konsistenz- oder Redundanzbedingungen 6 basieren darauf, dass in zu unterschiedlichen Angulationen (Projektionsrichtungen des Aufnahmesystems bezüglich des Untersuchungsobjekts) gehörigen Projektionsbildern Redundanzen in den aufgenommenen Daten vorliegen.

In der o.g. Veröffentlichung wird eine derartige Konsistenzbedingung verwendet, um Patientenbewegung zu kompensieren, also die Projektionsgeometrie (definiert durch eine Liste von Projektionsmatrizen) nach der Akquisition der Projektionsbilder an die aufgetretene Patientenbewegung anzupassen, so dass das resultierende Rekonstruktionsergebnis möglichst wenige Bewegungsartefakte aufweist. Der Ansatz in der Erfindung verwendet im Gegensatz dazu die Konsistenz- oder Redundanzbedingung, um geeignete Projektionsbilder zu identifizieren und aus diesen dann das sich bewegende Untersuchungsobjekt im gewünschten Bewegungszustand zu rekonstruieren. Daher darf beim vorliegenden Verfahren keine signifikante Kontrastmitteldynamik in den Projektionsbildern sichtbar sein, da diese Inkonsistenzen verursachen und so Verletzungen der Konsistenzbedingung implizieren würde; dies gilt auch für den Fall dass sich das Untersuchungsobjekt gar nicht bewegt.

Die Konsistenz- oder Redundanzbedingung kann auch bereits zuvor ermittelt worden sein und nun aus einer Speichereinheit abgerufen werden.

In einem vierten Schritt 4 werden durch Anwendung der Konsistenz- oder Redundanzbedingung auf die aufgenommenen Projektionsbilder der Serie, also durch ein Filterverfahren, weitere Projektionsbilder Pᵢ₁, Pᵢ₂, ... Pᵢ₍ₖ₋₁₎, Pᵢₖ ermittelt, welche den ersten Bewegungszustand abbilden. Diese weiteren Projektionsbilder Pᵢ₁, Pᵢ₂, ... Pᵢ₍ₖ₋₁₎, Pᵢₖ werden retrospektiv aus dem Datensatz ermittelt bzw. errechnet, ohne weitere Daten hinzuziehen zu müssen, alleine durch eine Filterung unter Verwendung der Konsistenz- oder Redundanzbedingung. Die ermittelten Projektionsbilder Pᵢ₁, Pᵢ₂, ... Pᵢ₍ₖ₋₁₎, Pᵢₖ, mindestens zwei, im Allgemeinen eine Vielzahl von Projektionsbildern, bilden alle den zuvor ausgewählten Bewegungszustand ab. Wenn nur sehr wenige Projektionsbilder ermittelt werden können, kann es sinnvoll sein, einen vorgebbaren Toleranzschwellwert der Konsistenz- oder Redundanzbedingung bezüglich des Bewegungszustands zu vergrößern, so dass eine größere Anzahl von Projektionsbildern gefunden werden kann. Dies kann zwar zu einer Bewegungsunschärfe in den späteren Volumenbildern führen, steigert aber durch eine höhere Anzahl zu rekonstruierender Projektionsbilder die generelle Volumenbildqualität durch Minimierung von Unterabtastungsartefakten. Es kann vorgesehen sein, dass der Toleranzschwellwert für einen Nutzer auswählbar ist, also durch eine Eingabe höher, aber auch niedriger, ausgewählt wird.

Aus den derart identifizierten Projektionsbildern wird in einem fünften Schritt 5 das Untersuchungsobjekt im ausgewählten Bewegungszustand rekonstruiert. Dazu werden das erste Projektionsbild Pᵢ und die ermittelten Projektionsbilder Pᵢ₁, Pᵢ₂, ... Pᵢ₍ₖ₋₁₎, Pᵢₖ, welche alle den ersten Bewegungszustand abbilden, zu einem ersten Volumenbild 7 rekonstruiert. Die Rekonstruktion wird z.B. mit einem aus der Literatur bekannten iterativen Rekonstruktionsverfahren (algebraic reconstruction technique (ART), Simultaneous Algebraic Reconstruction Technique (SART)) durchgeführt, welches keine äquidistante Verteilung der Projektionsbilder entlang der Trajektorie des Scans erfordert.

Nach einer Ausführung der Erfindung kann das Verfahren auch mehrmals hintereinander durchgeführt werden und dadurch unterschiedliche Volumenbilder erzeugt werden, welche mehrere Bewegungszustände des Untersuchungsobjekts abbilden. So kann z.B. bei einem aufgenommenen Atemzyklus eine Vielzahl von Volumenbildern zwischen maximaler Exhalation bis zur maximalen Inhalation erzeugt werden. Diese Volumenbilder können anschließend zu einer Abfolge von Volumenbildern, also einer Art 3D-Videosequenz, verbunden werden.

Abgrenzung des erfindungsgemäßen Verfahrens: Es geht nicht um Ansätze wie das 4D-DSA Verfahren, bei welchem die Kontrastmitteldynamik (basierend auf einem Maskenlauf und einem Füllungslauf des C-Bogens) in einem als rigide angenommenen Gefäßsystem zeitaufgelöst approximativ rekonstruiert wird, was einer zeitaufgelösten Darstellung von Hochkontrastobjekten entspricht. Das erfindungsgemäße Verfahren funktioniert auch anders als die interventionelle dynamische Perfusionsbildgebung. Bei Letzterer geht es darum, die Kontrastmitteldynamik im Parenchym (v.a. im Gehirn beim akuten ischämischen Schlaganfall) zeitaufgelöst tomographisch zu rekonstruieren. Bewegungen des Patienten werden dabei allenfalls mittels rigider 2D/2D Registrierung korrigiert.

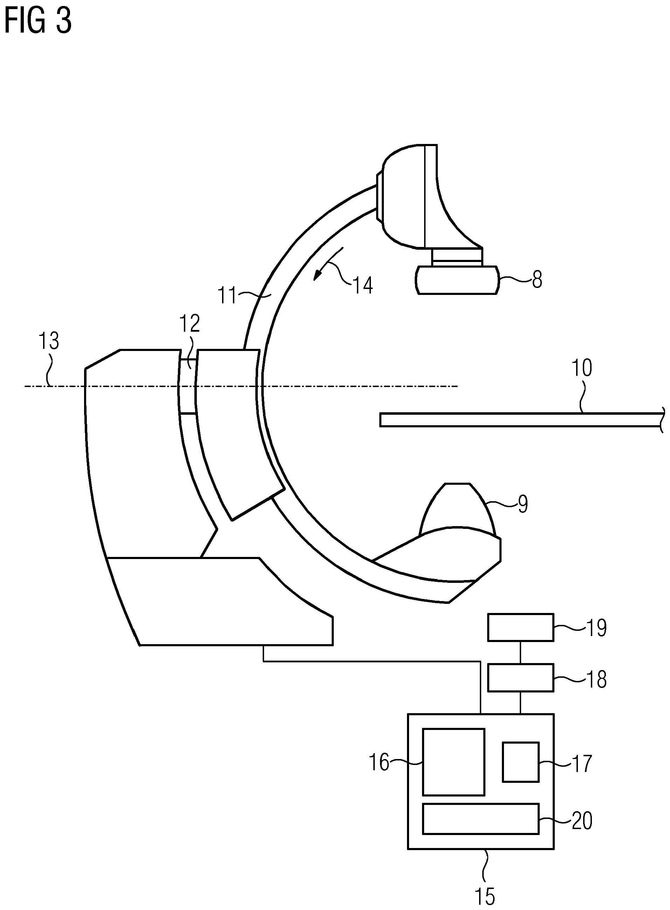

In der FIG 3 ist ein beispielhaftes Röntgenbildgebungssystem mit einem C-Bogen 11 gezeigt, welches zur Durchführung des Verfahrens geeignet ist. An dem C-Bogen 11 sind eine Röntgenquelle 9 und ein Röntgendetektor 8 angeordnet. Der C-Bogen 11 kann in mehreren Ebenen bewegt werden, unter anderem kann er in Richtung des Pfeils 14 verschoben werden und um die Achse 13 rotiert werden. Bei dieser Rotation um ein auf dem Patiententisch 10 angeordnetes Untersuchungsobjekt kann eine Vielzahl von Projektionsbildern des Untersuchungsobjekts aus unterschiedlichen Projektionsrichtungen (Angulationen) aufgenommen werden. In der Aufhängung 12 ist beispielhaft ein Schleifring angeordnet, durch den der C-Bogen 11 in einer Endlosrotation um die Achse 13 rotiert werden kann. Bei bisherigen Röntgenbildgebungssystemen ohne Schleifring war dies nur maximal bis zu einer Umdrehung möglich, dann musste wieder zurückgedreht werden. Mit der Einführung eines Schleifrings im Rotationsteil der Angulation kann der C-Bogen 11 uneingeschränkt rotieren. Unterschiedliche konstante Rotationsgeschwindigkeiten sind problemlos möglich. Das Bewegungsfeld des Röntgenbildgebungssystems ist durch den Einsatz des Schleifringes besonders glatt.

Das Röntgenbildgebungssystem weist eine Systemsteuerung 15 zur Ansteuerung, außerdem ein Bildbearbeitungssystem 15 mit einer Recheneinheit 16 zur Ermittlung der Konsistenz- oder Redundanzbedingung, zur Bearbeitung von Projektionsbildern und zur Rekonstruktion der Projektionsbilder zu Volumenbildern auf. Es kann sich zum Beispiel um einen Bildbearbeitungscomputer handeln. Außerdem weist das Röntgenbildgebungssystem noch eine Speichereinheit 17, eine Eingabeeinheit 18, z.B. eine Tastatur oder einen Touchbildschirm, und eine Anzeigeeinheit 19, z.B. einen Monitor oder ein Touchbildschirm, auf.

Alternativ kann das Röntgenbildgebungssystem auch von einem Computertomographiesystem gebildet werden, auch hier ist eine Endlosrotation möglich.

Generell sind auch andere Röntgenbildgebungssysteme zur Durchführung des Verfahrens möglich. Wichtig ist, dass das System eine konstante Rotationsdynamik aufweist und dadurch möglichst geringe mechanische Instabilitäten bei der Aufnahme der Serie von Projektionsbildern zeigt, damit die Verletzung der Konsistenz- oder Redundanzbedingung lediglich durch die Bewegung des Untersuchungsobjekts zu Stande kommt.

Die Erfindung lässt sich in folgender Weise kurz zusammenfassen: Für eine vereinfachte zeitaufgelöste 3D-Röntgenbildgebung mit hohem Weichteilkontrast mittels eines C-Bogen-Röntgenbildgebungssystems ist ein Verfahren zur Erstellung eines Volumenbildes eines sich bewegenden Untersuchungsobjekts in einem ausgewählten Bewegungszustand aus einer Serie von Projektionsbildern, welche während einer Rotationsbewegung eines Aufnahmesystems um das Untersuchungsobjekt mit jeweils einer Vielzahl von Projektionsbildern aus unterschiedlichen Projektionsrichtungen aufgenommen wurde, mit den folgenden Schritten, vorgesehen: Bereitstellung der Serie von Projektionsbildern (P₁, P₂ ...P_{N}), Auswahl eines ersten Bewegungszustands insbesondere durch Auswahl eines ersten Projektionsbildes, das den ersten Bewegungszustand abbildet, Bereitstellung oder Ermittlung einer Konsistenz- oder Redundanzbedingung, Identifikation weiterer Projektionsbilder, die den ersten Bewegungszustand abbilden, durch Anwendung der Konsistenz- oder Redundanzbedingung auf die Projektionsbilder der Serie, und Rekonstruktion der identifizierten Projektionsbilder und des ersten Projektionsbildes zu einem rekonstruierten Volumenbild. Das erfindungsgemäße Verfahren funktioniert algorithmisch und rein bildbasiert.

## Patentansprüche

1. Verfahren zur Erstellung eines Volumenbildes eines sich bewegenden Untersuchungsobjekts in einem ausgewählten Bewegungszustand aus einer Serie von Projektionsbildern, welche während einer Rotationsbewegung eines Aufnahmesystems um das Untersuchungsobjekt mit jeweils einer Vielzahl von Projektionsbildern aus unterschiedlichen Projektionsrichtungen aufgenommen wurde, mit den folgenden Schritten:
• Bereitstellung der Serie von Projektionsbildern (P₁, P₂ ... P_{N}),
• Auswahl eines ersten Bewegungszustands insbesondere durch Auswahl eines ersten Projektionsbildes (Pᵢ), das den ersten Bewegungszustand abbildet,
• Bereitstellung oder Ermittlung einer Konsistenz- oder Redundanzbedingung (6),
• Identifikation weiterer Projektionsbilder (Pᵢ₁, Pᵢ₂, ... Pᵢ₍ₖ₋₁₎, Pᵢₖ), die den ersten Bewegungszustand abbilden, durch Anwendung der Konsistenz- oder Redundanzbedingung auf die Projektionsbilder (P₁, P₂ ... P_{N}) der Serie, und
• Rekonstruktion eines Volumenbilds (7) aus den identifizierten Projektionsbildern (Pᵢ₁, Pᵢ₂, ... Pᵢ₍ₖ₋₁₎, Pᵢₖ) und dem ersten Projektionsbild (Pᵢ).

2. Verfahren nach Anspruch 1, wobei die Serie von Projektionsbildern während zumindest einem halben Rotationslauf des Aufnahmesystems um das Untersuchungsobjekt aufgenommen wurde.

3. Verfahren nach Anspruch 1 oder 2, wobei die Rekonstruktion mittels eines iterativen Rekonstruktionsverfahrens, insbesondere eines ART oder SART, durchgeführt wird.

4. Verfahren nach einem der vorangehenden Ansprüche, wobei mehrere Volumenbilder eines Untersuchungsobjekts in mehreren, insbesondere aufeinander abfolgenden, ausgewählten Bewegungszuständen ermittelt werden.

5. Verfahren nach Anspruch 4, wobei die Volumenbilder zu einem zeitlich abhängigen Bilddatensatz zusammengefügt werden.

6. Verfahren nach einem der vorangehenden Ansprüche, wobei die Auswahl des ersten Bewegungszustands automatisch oder manuell durch eine Eingabe eines Benutzers durchführbar ist.

7. Verfahren nach einem der vorangehenden Ansprüche, wobei die Konsistenz- oder Redundanzbedingung (6) einen vorgebbaren Toleranzschwellwert bezüglich des Bewegungszustands aufweist.

8. Röntgenbildgebungssystem zur Durchführung des Verfahrens nach einem der Ansprüche 1 bis 7, aufweisend ein Aufnahmesystem mit einem Röntgendetektor (8) und einer Röntgenquelle (9), die an einer Halterung angeordnet sind, welche Halterung dazu ausgebildet ist, um ein Untersuchungsobjekt zu rotieren und dabei eine Vielzahl von Projektionsbildern aus unterschiedlichen Projektionsrichtungen aufzunehmen, sowie aufweisend eine Systemsteuerung (15) zur Ansteuerung des Röntgenbildgebungssystems, eine Recheneinheit (16) zur Ermittlung der Konsistenz- oder Redundanzbedingung und ein Bildbearbeitungssystem (15) zur Bearbeitung von Projektionsbildern und zur Rekonstruktion der Projektionsbilder zu Volumenbildern.

9. Röntgenbildgebungssystem nach Anspruch 8, wobei die Halterung von einem C-Bogen (11) gebildet wird und der C-Bogen (11) mittels einer Schleifringkonstruktion aufgehängt ist, so dass eine Endlosrotation des C-Bogens (11) ausführbar ist.

10. Röntgenbildgebungssystem nach Anspruch 8, welches von einem Computertomographiesystem gebildet wird.

11. Röntgenbildgebungssystem nach einem der Ansprüche 8 bis 10, aufweisend eine Speichereinheit (17) zur Speicherung von Bilddaten und/oder Berechnungsdaten.

12. Röntgenbildgebungssystem nach einem der Ansprüche 8 bis 11, aufweisend eine Eingabeeinheit (18) zur Entgegennahme einer Benutzereingabe.
